# EUROPEAN PATENT SPECIFICATION

(11) **EP 2 774 626 B1**
(45) Date of publication and mention of the grant of the patent: **01.11.2017**
(21) Application number: 12198386.0
(22) Date of filing: 20.12.2012
(51) Int. Cl.: A61L 2/07, A23C 3/037, A23L 3/015

(54) **Apparatus and method for the sterizalisation, degassing and concentration of viscous products**
Apparat und Verfahren zum Sterilisieren, Entgasen und Aufkonzentrieren viskoser Erzeugnisse
Appareil et procédé pour la stérilisation, le dégazage et la concentration de produits visqueux

(30) Priority: 13.12.2012 US 201213713760
(43) Date of publication of application: 10.09.2014
(73) Proprietor: SPX APV Danmark A/S, 8600 Silkeborg (DK)
(72) Inventor: Coelho, Gil, 27110 Epreville près le Neubourg (FR); Braud, Bernard, 27240 Les Essarts (FR)
(74) Representative: Collet, Alain

(56) References cited:
- EP-A1- 0 054 960
- EP-A2- 0 923 879
- WO-A1-2006/024762
- US-A- 5 992 169

## Description

### TECHNICAL FIELD

The present invention relates to apparatuses and methods for sterilizing viscous fluids such as, for example, cosmetic products, drugs, vegetal and animal extracts, and/or food products. Certain embodiments of the present invention also relate to an apparatus for degassing or concentrating viscous fluids. This invention relates generally to devices, systems, and methods for processing viscous fluids.

### BACKGROUND ART

The industrial production of food has faced for years the issue of aseptic production and packaging. The aim of such production and packaging is to extend shelf life of products, to preserve nutritional qualities for an extended period of time and to kill germs potentially harmful to health. Government regulations have rapidly constrained the range of options available to extend product shelf life and specifically banned inclusion of many preservatives in foodstuffs destined for human ingestion.

Heat treatment of liquid food products for the purpose of sterilization is a commonly used industrial process. To achieve rapid heating up to high temperatures, typically exceeding 100°C, steam is employed. The heating may take place either directly or indirectly. In indirect heating, use is made of different types of heat exchangers. In direct heating, steam is directly incorporated into the product. A direct heating sterilization process, well known for dairy products, is commonly referred to as the UHT process, which stands for *Ultra Heat Treatment* or *Ultra High Temperature;* an appropriate control of both temperature level and exposure duration of the heat treatment allowing the extermination of those microorganisms which are found in dairy products.

There are two types of steam incorporation processes, injection and infusion. With injection, steam is injected into the product in a closed system, whereas infusion implies that the product is finely sprayed and caused to pass through a space filled with steam. In both cases, the supplied steam rapidly and efficiently heats the product to the desired temperature and the product is then kept at this temperature during a given predetermined exposure duration. The supplied steam must thereafter be removed from the product in order to avoid diluting it.

This normally takes place by evaporative cooling, commonly referred as flash-cooling, in a vacuum chamber. During the process, the steam is released and condensed at the same time as the product is cooled down to a similar temperature it had before the heat treatment. The evaporative cooling usually takes place by feeding the steamed product, under pressure, into a vacuum chamber. When the product enters the vacuum chamber, the liquid boils, the steam is released and rises upwards in the chamber while the product accumulates in the lower region of the chamber. Thus cooled, the product may be tapped off from the lower region of the chamber. The steam which leaves the product together with incondensable gases is to be condensed in order for it to be able to be run off to an outlet. Document US 59992169 discloses a system of vacuum cooling of crop products (e.g. vegetables). In the system, the products to be cooled are placed in bins , placed in the vacuum chamber . To improve the efficiency of the cooling, water is injected into the upper part. Document EP 0923879 discloses a device for the cooling by vacuum of a viscous liquid comprising solid pieces (e.g. food preparation). In the process, the fluid to be cooled is injected via an inlet duct at the top of a vacuum column. The fluid collected at the bottom of the column is periodically transferred to a temporary storage tank through a valve. The fluid is then evacuated via the outlet duct by means of the pump. Document EP 0054960 discloses a device for producing sweets from a solution of water, sugar and glucose. In the process, the aqueous solution is first kept at high temperature in an enclosure, before being cooled through a first evaporation chamber and then a second evaporation chamber communicating by gravity with the first. The solution then flows by gravity to a third chamber. In order to extract the solution from the bottom of the third chamber. Document WO2006/024762 discloses a process for cooling plants, in particular grapes for the production of wine. The process comprises, firstly, a heating step in a heating chamber. The grapes are then introduced into a first vacuum chamber. The vaporization causes a destructuring of the grapes. The resulting harvest is recovered at the bottom of the first chamber and then transferred by means of a volumetric pump to a second vacuum enclosure.

The cosmetic industry is facing a similar issue. As one example, skincare and cosmetic creams are meant to be used multiple times, for a use life extended to months. To address that issue, the cosmetic industry includes various preservatives, such as parabens, which prevent the growth of fungi and bacteria. A number of studies highlighted the potential harmfulness of such preservatives to human health. Other compounds have been investigated as paraben substitutes, but show lower efficiency and/or still carry the risk of long term impact on human health. Consequently, regulations are being more and more restrictive about preservatives integration into cosmetic products.

As an alternative route to incorporating preservatives, it was thought to sterilize cosmetic products. Several methods and devices were investigated. In particular, United States Patent Application US 2009/0148343 discloses the sterilization of a cosmetic product based upon indirect heating, by means of circulating the product through a circuit traversing heating and cooling baths.

However, the state of the art apparatus and methods suffer from limited efficiency of the sterilization process. More specifically, viscous products show a poor heat penetration and the heating and cooling durations are long, usually above several seconds. It implies a high shear rate during the process which may ultimately modify product viscosity.

### SUMMARY OF THE INVENTION

The incorporation of steam is a good solution to bring heat to a viscous product. However, because of the specific nature of cosmetic products, their sterilization through a direct heating process is a challenge which requires solving several technical problems. The heating process should be fast and efficient to bring the heat to the fluid without damaging it. Also the cooling process should be fast and efficient to minimize or eliminate the denaturization of sensitive components such as proteins, vitamins, etc... To maintain a product emulsion, as often encountered in the cosmetic industry, and to avoid phase separation (e.g., aggregation and/or precipitation), it is also often desirable, and sometimes required, to bring the final product, after sterilization, to low temperature, typically in the range of between 40°C and 90°C, which generally means high viscosity of the cosmetic product.

A constraint previously mentioned is to avoid shearing of the fluid to preserve the viscosity of the output product to a level substantially equivalent or at least similar to that of the input product. Also, the water content after the evaporative cooling step should often preferably be kept in a controlled range.

The evaporative cooling step is a specific challenge when applying a direct heating sterilization process to viscous products: On a first hand, a low vacuum pressure is usually required to allow efficient water evaporation from the viscous fluid, and on a second hand, the high viscosity of the fluid means low hydrostatic pressure at the bottom of the vacuum vessel. For these reasons, the state of the art evaporative cooling devices are not adapted to viscous fluid. In most case, they result in obstruction of the pump, potential cavitation issue, and an accumulation of fluid in the evaporative cooling device.

It is one object of the present invention to cope with the disadvantages of the state of the art by providing an apparatus for the sterilization of a viscous fluid through a direct heating approach.

To this end, certain embodiments of the present invention relate to an evaporative cooling device for a viscous fluid including a vacuum chamber, having an upper part wherein the viscous fluid is injected, a lower part extending downwardly from the upper part wherein the viscous fluid is collected by gravitational force, and an outlet pipe located at bottom of lower part, through which the viscous fluid is extracted. The evaporative cooling device further includes an extraction device, having a primary pump, fed with viscous fluid from the lower part through the outlet pipe, and a feeding device, located inside the lower part of vacuum chamber, which pushes the viscous fluid from the lower part towards the primary pump, through the outlet pipe.

Certain embodiments of the present invention also relate to an apparatus for the sterilization of a viscous fluid, including a direct heating device, wherein the viscous fluid is heated up to a temperature range of between 120°C and 155°C by incorporating steam into the viscous fluid; a high temperature circuitry, fed with heated fluid from the direct heating device, wherein the heated fluid is maintained in the temperature range of between 120°C and 155°C for a predetermined residence time, configured to allow sterilization of the viscous fluid; and the evaporative cooling device as previously described, fed with the heated fluid from the high temperature circuitry, wherein the heated fluid is cooled to a temperature of between 40°C and 90°C by steam evaporation in the vacuum chamber set at a pressure of between 0.05 and 1 bar (absolute pressure).

Additionally, certain embodiments of the present invention relate to a method for the sterilization of a viscous fluid, wherein the viscous fluid circulates throughout a sterilization circuit including the evaporative cooling device previously described, which method includes the (sometimes successive) steps of heating quickly, (e.g., in less than 1 second), the fluid by incorporation of steam in the fluid, to a temperature range of between 120°C and 155°C; maintaining fluid temperature in the range of between 120°C and 155°C for a predetermined residence time, to allow sterilization of the fluid; cooling down quickly, (e.g., in less than 1 second), the fluid by steam evaporation, down to a temperature range of between 40°C and 90°C, in the evaporative cooling device.

It is another object of certain embodiments of the present invention to propose a solution for the degassing or water concentration adapted to a viscous fluid.

To this end, certain embodiments of the present invention relate to an apparatus for the degassing of a viscous fluid including the evaporative cooling device previously described. Certain embodiments of the present invention also relate to an apparatus for the concentration of water content of a viscous fluid including an evaporative cooling device previously described.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE ACCOMPANYING DRAWINGS

Other features and advantages of the present invention will become clear from the following detailed description of the non limitative embodiments of the invention, referring to the accompanying Drawings representing schematically the apparatus of the invention. In the accompanying Drawings:
Fig. 1 is a flow diagram according to a first embodiment of an apparatus for the sterilization of a viscous fluid; and
Fig. 2 is a flow diagram according to a second embodiment of an apparatus for the sterilization of a viscous fluid; and
Fig. 3 is a flow diagram according to a third embodiment of an apparatus for the sterilization of a viscous fluid; and
Fig. 4 is a side elevation of an evaporative cooling device of the invention; and
Fig. 5 is a side elevation, partly in section, of an extraction device of the invention; and
Fig. 6 is a scheme of a feeding device for the extraction device of the invention.

### DESCRIPTION OF PREFERRED EMBODIMENTS

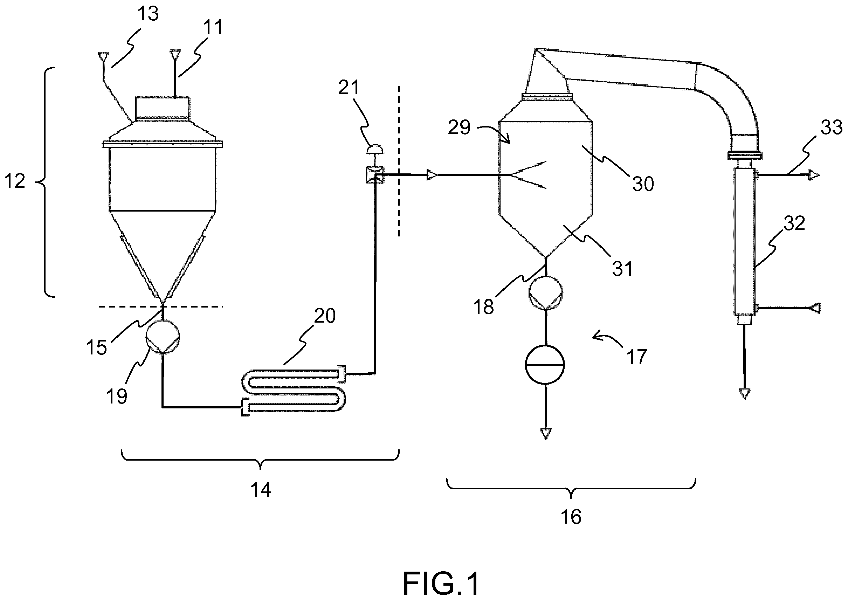

A flow diagram of a first embodiment of an apparatus for the sterilization of a viscous fluid is shown in detail in Fig. 1. The apparatus is designed for cosmetic products, such as skincare creams or lotions, including emulsion products. However, the present invention is not limited to cosmetic products, but more generally covers fluids of high viscosity. Other viscous fluids for which the present invention is adapted include, for example, drugs, vegetal and animal extracts, and food products. Certain embodiments of the present invention aim at sterilizing any type of viscous fluids, preferably having a static viscosity exceeding 1500 cP at a temperature between 40°C and 90°C. It has been shown that certain embodiments of the present invention are especially adapted to fluid for which a static viscosity exceeding 1500 cP is measured in this temperature range and after an evaporative cooling step, e.g. after sterilization based upon direct heating.

To allow for a fast heating and fast cooling of the viscous fluid to be sterilized, the certain embodiments of the present invention relate to the method of steam incorporation in the fluid. The method often includes at least three main successive steps, often performed successively in the order listed below :
- heating quickly the viscous fluid by incorporation of steam into the fluid,
- maintaining the heated fluid at high temperature to sterilize the fluid,
- quickly cooling the fluid by steam release.

In a first embodiment of the present invention as shown in Fig 1, the apparatus for the sterilization of a viscous fluid 11 includes:
- a direct heating device 12, wherein the viscous fluid 11 is heated up by incorporating steam 13 in the viscous fluid 11, to a temperature range of between 120°C and 155°C,
- a high temperature circuitry 14, fed with heated fluid from the direct heating device 12, wherein the heated fluid 15 is maintained in a temperature range of between 120°C and 155°C for a predetermined residence time to allow sterilization of the viscous fluid,
- an evaporative cooling device 16, fed with heated fluid from the high temperature circuitry 14, wherein steam 13 is released from the heated fluid by evaporation, cooling it down to a temperature of between 40°C and 90°C.

According to certain embodiments of the invention, the inlet viscous fluid 11 is preheated by an indirect heating device located upstream of the direct heating device 12 (not represented on the figure) to a temperature of between 40°C and 90°C before it is introduced into the direct heating device 12. In the first embodiment as shown on figure 1, the heating device 12 is a steam infusion device. It includes a chamber fed with steam 13, having an upper cylindrical part and a lower conical part. The inlet fluid 11 is introduced in the upper cylindrical part and finely divided, for example by means of a dedicated perforated plate. Steam is incorporated into the fluid which is sprayed in the chamber. The lower conical part collects the heated fluid before transferring it to the high temperature circuitry 14.

An efficient distribution of the steam flow, together with an efficient distribution of fluid inside the chamber, lead to an efficient heating of the viscous fluid. A temperature in the range of between 120°C and 155°C is reached in less than 1 second. Such values are very attractive for the preservation of the viscous fluid characteristics, especially when compared to state of the art apparatuses based upon indirect heating.

According to certain embodiments of the present invention, the temperature of the heated fluid 15 is then maintained in the same range throughout the high temperature circuitry 14. In the first embodiment as shown in Fig. 1, the high temperature circuitry 14 includes successively:
- a centrifugal pump 19, fed with heated fluid from the direct heating device, configured to ensure a fluid pressure of between 2 and 10 bars (absolute pressure),
- a holding tube 20, configured to maintain the heated fluid in a temperature range of between 120°C and 155°C for a predetermined residence time to allow for sterilization of the viscous fluid,
- a relief valve 21, configured to feed the evaporative cooling device 16 with the heated fluid 15, and to ensure a fluid pressure of between 2 and 10 bars (absolute pressure) upstream of the relief valve (21). According to certain embodiments of the present invention, pressure of the heated fluid downstream the relief valve is between 0.05 and 1.5 bars (absolute pressure).

The above design and physical values of the high temperature circuitry 14 are not limitative of the present invention. To achieve an expected level of microbial load or denaturation of a given molecule, one may adapt temperature and exposure duration of the fluid passing through the high temperature circuitry. For that matter, other devices and physical values may be selected.

Subsequently, the fluid is cooled down in the evaporative cooling device 16, which includes a vacuum chamber 29, having an upper cylindrical part 30 and a lower conical part 31 extending downwardly from the upper part 30. In the upper part 30 is injected the heated fluid 15. The fluid is then cooled down by evaporating, at least partially, the steam added to the fluid during the direct heating step. The lower part 31 collects the cooled down fluid by gravitational force. Depending on the vacuum level and the fluid inlet and outlet temperatures, the amount of evaporated water can be adjusted. It is also expected that the water content of the viscous fluid after sterilization, although in the same range as before sterilization, may be voluntarily modified. This could be the case when the evaporative cooling device according to certain embodiments of the invention is used in an apparatus dedicated to the concentration of water of a viscous fluid. This application will be further described in the following. The pressure of vacuum chamber is between 0.05 and 1 bars (absolute pressure). A flash cooling duration of less than one second is achieved to cool down the fluid to a temperature of between 40°C and 90°C. Such values are very attractive for the preservation of the viscous fluid characteristics, as they limit the product exposure to elevated temperature.

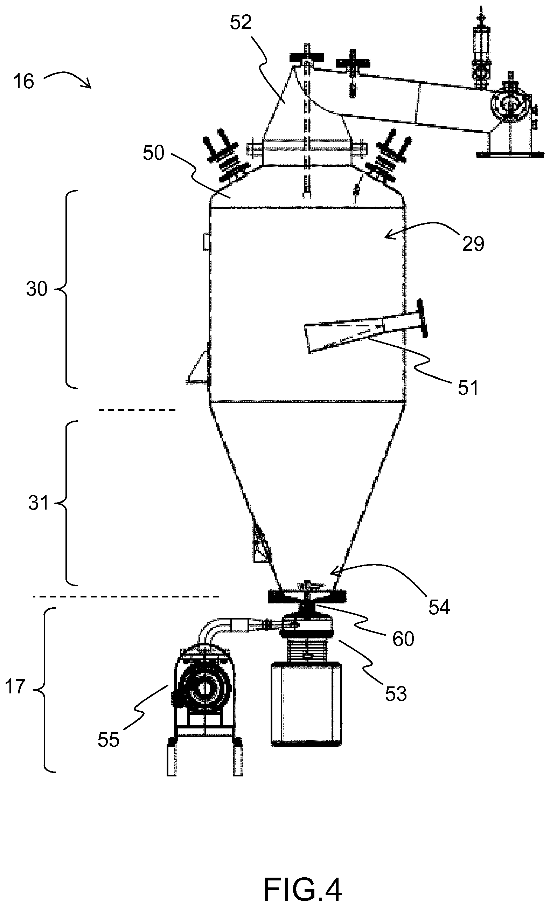

However, the high viscosity of the cooled down fluid 18 at bottom of the lower part 31 of vacuum chamber 29 may make it difficult to extract the fluid out of the evaporative cooling device 16. Direct pumping could result in obstruction of the pump, potential cavitation issue, and an accumulation of fluid in the evaporative cooling device. To address these potential issues, the evaporative cooling device according to certain embodiment of the present invention further includes an extraction device 17 to extract the cooled down fluid from the lower part 31 and to ensure its transportation to a downstream circuit, such as for example a sterile packaging unit (not represented on the figure). The extraction device 17 is further described below, with figures 4, 5 and 6. The evaporative cooling device 16 also includes a condenser 32 into which the steam released from the fluid is condensed to water, using a cooling liquid 33.

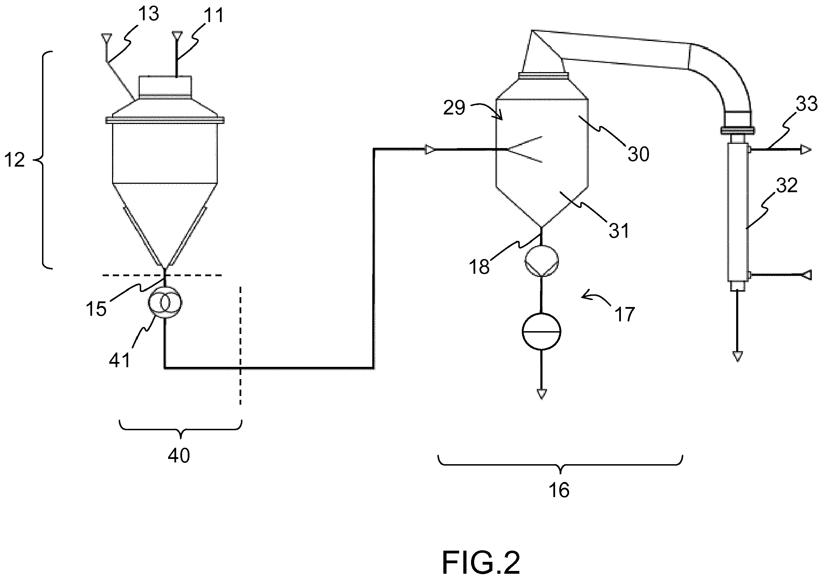

A flow diagram of a second embodiment of an apparatus for the sterilization of a viscous fluid is shown in detail in Fig. 2. The second embodiment involves a number of components which have been described in Fig 1. The same numbers on the figures refer to the same components; these components are not detailed again below.

The second embodiment as shown in Fig. 2 includes a direct heating device 12, a high temperature circuitry 40 and an evaporative cooling device 16. The high temperature circuitry 40 differs from the first embodiment. In this embodiment, it includes a positive displacement pump 41, fed with heated fluid from the direct heating device, and configured to ensure a fluid pressure of between 2 and 10 bars (absolute pressure) upstream of the positive displacement pump 41. This second embodiment is particularly suited for when very short residence time of the fluid is expected; the positive displacement pump acting as a brake to control back pressure between the circuitry connected to the heating device and the circuitry connected to the evaporative cooling device.

Although it is not represented in Fig. 2, certain embodiments of the present invention also include adding a holding tube to the high temperature circuitry 40. A similar holding tube as described in figure 1, and referred holding tube 20, may be added upstream or downstream the positive displacement pump 41.

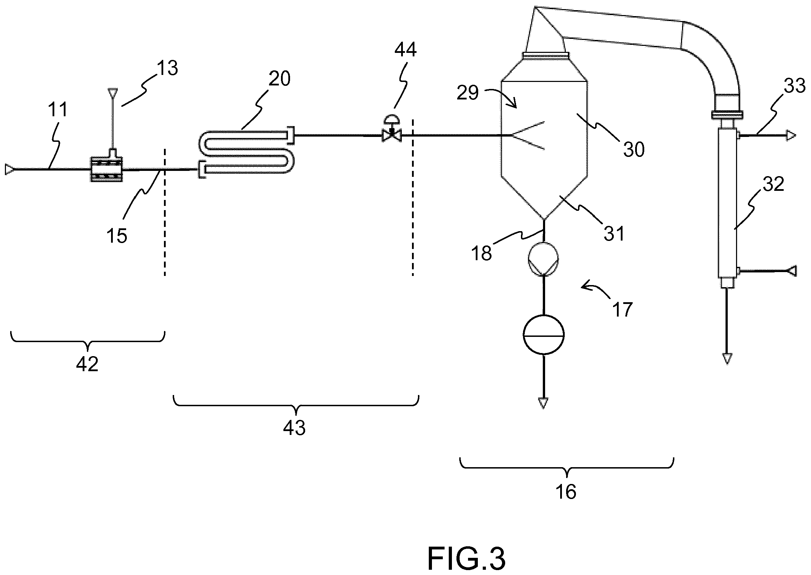

A flow diagram of a third embodiment of an apparatus for the sterilization of a viscous fluid is shown in detail in Fig. 3. The third embodiment involves a number of components which have been described in Fig. 1 or Fig. 2. The same numbers on the figures refer to the same components; these components are not detailed again below.

The third embodiment as shown in Fig. 3 includes a direct heating device 42, a high temperature circuitry 43 and an evaporative cooling device 16. The direct heating device 42 and the high temperature circuitry 43 differ from the first and second embodiment. In this embodiment, the direct heating device 42 is a steam injector device. The inlet viscous liquid 11, having a temperature in the range of between 40°C and 90°C, for example thanks to an indirect preheating step (not represented on the figure), is brought to pass through a steam injection nozzle in which the viscous fluid 11 is mixed with steam 13. The incorporation of steam heats up the fluid to a temperature in the range of between 120°C and 155°C in less than 1 second. At the outlet of the direct heating device 42, the heated fluid 15, under pressure in the range of 2 to 10 bars (absolute pressure), feeds the high temperature circuitry 43.

In the third embodiment, the steam injection device is configured to manage pressure of the outlet heated fluid 15, so that the high temperature circuitry 43 may not require an additional pump. Consequently, the high temperature circuitry 43 includes a holding tube 20 and a control valve 44, configured to control flow rate and pressure of the heated fluid. As previously mentioned, typical values of pressure for the heated fluid injected into the flash cooling device are between 0.05 and 1.5 bars (absolute pressure). The design of the high temperature circuitry adapted to steam injection is not limited to the design shown on Fig. 3. Other designs are possible. As an example the high temperature circuitry 14 and 40, as shown on Fig. 1 and Fig. 2, are considered.

A more detailed layout of the evaporative cooling device according to certain embodiments of the present invention is given in Fig. 4. The evaporative cooling device 16 includes a vacuum chamber 29, fed with viscous fluid having a temperature in the range of between 120°C and 155°C, having an upper part 30 and a lower part 31. In the upper part 30, having a cylindrical shape, steam is released from the heated fluid by evaporation, cooling it down to a temperature of between 40°C and 90°C. In the lower part 31, having a conical shape and extending downwardly from the upper vacuum chamber 30, the cooled down fluid 18 is collected by gravitational force. The lower part 31 includes an outlet pipe 60 through which the cooled down fluid 18 is extracted. The evaporative cooling device 16 also includes a top cover 50 onto which is located a released steam pipe 52. The evaporative cooling device 16 further includes at least one fluid inlet 51 located on the longitudinal wall of the cylindrical upper part 30 wherein the viscous fluid is injected. The fluid inlet 51 is not limited to be located in cylindrical upper part, but may also be positioned in lower part 31. The present invention is not limited to a vacuum chamber having a cylindrical upper part and a conical lower part. In some embodiments, the vacuum chamber may have a conical shape for both the upper and lower parts. In yet other embodiments, the vacuum chamber may include an upper part and/or a lower part having an ovoid shape.

The heated fluid 15 is injected through the inlet 51 into the upper part 30. The gap of pressure and temperature between the incoming fluid and the vacuum chamber 29, leads to release the steam from the viscous fluid. The steam rises upwards in the chamber to the released steam pipe 52, while the fluid falls down and accumulates at bottom of the lower part 31. The released steam collected together with incondensable gases by the released steam pipe 52 is condensed in the condenser 32 previously described.

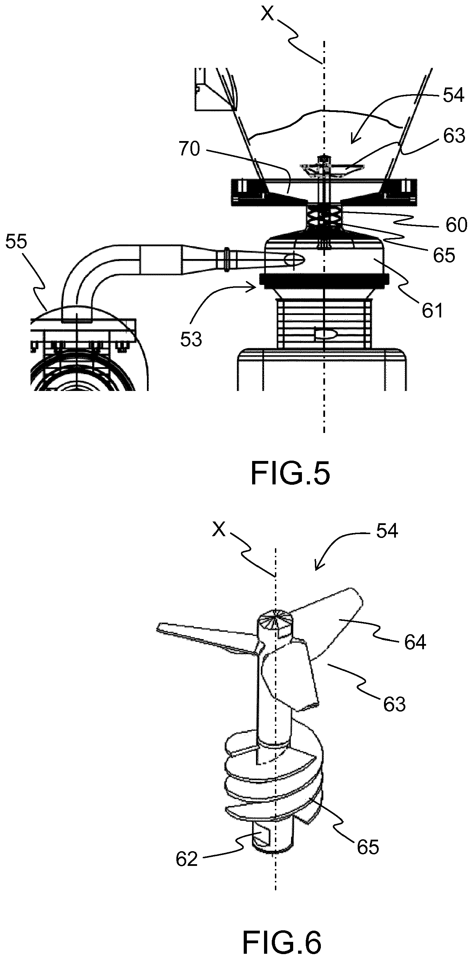

Because of fluid viscosity and vacuum level in the evaporative cooling device, the viscous fluid exerts a limited hydrostatic pressure on the lower part of the vacuum chamber. Gravitation force does not allow for an efficient passive feeding of a pump located at bottom of the lower part 31. Such direct pumping would lead to fluid accumulation in the vacuum chamber. To overcome the cooled down fluid viscosity, certain embodiments of the invention relate to an evaporative cooling device having an extraction device 17 which apply mechanical force to push, in an active manner, the fluid collected at bottom of lower part 31, towards a primary pump. According to certain embodiments, the extraction device 17 includes a primary pump 53, fed with the cooled down fluid 18 from the lower part 31 through the outlet pipe 60. The extraction device 17 further includes a feeding device 54, located inside the lower part 31 of vacuum chamber 29, which pushes the cooled down fluid 18 from the lower part 31 towards the primary pump 53, through the outlet pipe 60. Further details of the extraction device 17 are shown on Fig. 5 and Fig. 6 and discussed below.

In a common industrial installation, the sterilization apparatus is followed by a packaging unit, for example an aseptic packaging unit. To overcome pressure losses of piping installation between the evaporative cooling device and the packaging unit, the extraction device 17 as shown on Fig 4. further includes a secondary pump 55, for example a positive displacement pump or a centrifugal pump, located downstream the primary pump 53 which ensures or at least promotes transportation of the cooled down fluid 18 from the primary pump 53 to a downstream circuit. The primary pump acts as a booster pump to the secondary pump; the secondary pump being designed to overcome pressure losses of a downstream circuit.

Details of yet another embodiment of the extraction device are shown in Fig. 5 and Fig. 6. In this embodiment, the primary pump 53 is a centrifugal pump having an impeller 61 and a driveshaft 62. The feeding device 54 includes a propeller 63 located inside the lower part 31 of vacuum chamber 29 and which rotation axis is aligned with the impeller rotation axis, referred as X axis on Fig. 5 and Fig. 6. The driveshaft 62 passes through the outlet pipe 60 and is linked to the propeller 63 so as to drive in rotation both the impeller 61 and propeller 63. The propeller 63 as shown on Fig. 6 includes three blades 64. Other propeller configurations including at least two blades are possible according to the present invention.

As a result to the above design, the cooled down fluid 18, accumulated at bottom of the lower part 31 of vacuum chamber 29, is pushed by the propeller 63 towards the primary pump 53. The mechanical action of the propeller blades on the fluid generates a local pressure allowing to efficiently feed the centrifugal pump, and avoiding cavitation issues faced when pump feeding relies only on hydrostatic pressure of the fluid and suction capability of the pump.

The feeding device 54 further includes an endless screw 65 linked to the driveshaft 62, located between the impeller 61 and the propeller 63, and at least partially, inside the outlet pipe 60. The endless screw rotation axis is aligned with the impeller rotation axis X. The driveshaft 62 is able to drive in rotation the impeller 61, the endless screw 65 and the propeller 63. The use of the endless screw 65 further improves the efficiency of the extraction device 17. Referring to the NPSH parameter known by the person skilled in the art, and standing for *Net Positive Suction Head,* the use of the endless screw is beneficial as it reduces the required NPSH value at the pump impeller.

In the embodiment as shown on Fig. 5, the lower part 31 of vacuum chamber 29 further includes a concave surface of revolution 70 around a substantially vertical axis, onto which surface 70, the cooled down fluid 18 accumulates. As shown on Fig.5, the outlet pipe 60 is located at the bottom of the surface 70. The rotation axis X of the driveshaft 62 is substantially aligned with the vertical axis of revolution of surface 70. Different types of concave surface of revolution are considered to be within the scope of the present invention. As shown on Fig. 5, the surface 70 may be provided by two successive conical portions. Other configurations include a paraboloid surface or a ferrule type surface. A benefit of such concave surface 70 is to avoid the bouncing effect of the fluid which would occur in a configuration where the fluid is pushed by propeller blades against a non concave surface, such as a simple conical surface.

Certain embodiment of the present invention also relate to a method for the sterilization of a viscous fluid, wherein the viscous fluid circulates throughout a sterilization circuit including an evaporative cooling device 16 as previously described, which method includes the following steps, which may be performed successively in the order listed below:
- heating quickly, in less than 1 second, the fluid by incorporation of steam 13 in the fluid, to a temperature range of between 120°C and 155°C,
- maintaining fluid temperature in the range of between 120°C and 155°C for a predetermined residence time, to allow sterilization of the fluid,
- cooling down quickly, in less than 1 second, the fluid by steam evaporation, down to a temperature range of between 40°C and 90°C, in the evaporative cooling device 16.

According to certain embodiments of the present invention, the method includes an initial step of preheating the fluid, by means of an indirect heating device, to a temperature of between 40°C and 90°C.

In another embodiment of the present invention, the method is applied to a viscous fluid having a static viscosity above 5000 cP at a temperature between 40°C and 70°C. The method includes the following steps, which may be performed successively in the order listed below:
- heating quickly, in less than 1 second, the fluid by incorporation of steam 13 in the fluid, to a temperature range of between 120°C and 155°C,
- maintaining fluid temperature in the range of between 120°C and 155°C for a predetermined residence time, to allow sterilization of the fluid,
- cooling down quickly, in less than 1 second, the fluid by steam evaporation, down to a temperature range of between 40°C and 70°C, in the evaporative cooling device 16,
and wherein the vacuum level of the vacuum chamber is between 0.05 and 0.4 bars (absolute pressure).

In the description above and the accompanying drawings, particularly through Fig. 4, 5 and 6, embodiments of an evaporative cooling device adapted to a viscous fluid have been described. The implementation of such evaporative cooling device for the sterilization of viscous products has been described, particularly through Fig. 1, 2 and 3. The use of the evaporative cooling device for sterilization purpose is not limitative to the present invention. It has been mentioned, for example, that the amount of evaporated water during the flash cooling step can be adjusted voluntarily, by tuning differently some parameters such as vacuum level or fluid inlet and outlet temperatures. Independently from sterilization purpose, the evaporative cooling device according to certain embodiments of the invention is very much adapted to a process aiming at concentrating water content of a viscous fluid. Typical examples of industrial process where concentration of a viscous fluid is desirable or required include the concentration of an emulsion cosmetic product, or of animal or vegetal extracts. As a result, certain embodiments of the present invention relate to an apparatus for the concentration of water content of a viscous fluid, including an evaporative cooling device having the previously described features.

In a similar approach, the flash cooling method can be implemented for degassing purpose. In a direct heating sterilization approach, steam is voluntarily incorporated into the fluid, before it is released in the flash cooling step. In another approach, the evaporative cooling device of certain embodiments of the invention may be implemented for its capability to extract gas contained into a viscous fluid. Typical examples of industrial process where degassing of a fluid is required include the degassing of sparkling beverage or of animal or vegetal extract. As a result, certain embodiments of the present invention relate to an apparatus for the degassing of a viscous fluid, characterized in that it includes an evaporative cooling device having the previously described features.

## Claims

1. An evaporative cooling device for a viscous fluidcomprising:
- a vacuum chamber (29), having an upper part (30) wherein the viscous fluid is injected, a lower part (31) extending downwardly from the upper part (30) wherein the viscous fluid is collected by gravitational force, and an outlet pipe (60) located at bottom of lower part (31), through which the viscous fluid is extracted; and
- an extraction device (17), comprising a primary pump (53), fed with viscous fluid from the lower part (31) through the outlet pipe (60), and a feeding device (54), located inside the lower part (31) of vacuum chamber (29), which pushes the viscous fluid from the lower part (31) towards the primary pump (53), through the outlet pipe (60),
**characterized in that**
the primary pump (53) comprises a centrifugal pump having an impeller (61) and a driveshaft (62), wherein the feeding device (54) comprises a propeller (63) located inside lower part (31), and wherein the driveshaft (62) passes through the outlet pipe (60) and is linked to the propeller (63) so as to drive in rotation both the impeller (61) and propeller (63).

2. The device according to claim 1, wherein the feeding device (54) further comprises an endless screw (65) linked to the driveshaft (62), located between the impeller (61) and the propeller (63), and at least partially inside the outlet pipe (60), and wherein the driveshaft (62) is able to drive in rotation the impeller (61), the endless screw (65) and the propeller (63).

3. The device according to claim 1 or 2, wherein the lower part (31) further comprises a concave surface of revolution (70) around a substantially vertical axis, onto which surface (70) the cooled down fluid accumulates.

4. The device according to any of the claims 1 to 3, wherein the propeller (63) comprises at least two blades (64).

5. The device according to any of the claims 1 to 4, wherein the extraction device (17) comprises a secondary pump (55) located downstream the primary pump (53) which ensures the viscous fluid transportation from the primary pump (53) to a downstream circuit.

6. The device according to claim 5, wherein the secondary pump (55) comprises a positive displacement pump or a centrifugal pump.

7. An apparatus for the sterilization of a viscous fluid, **characterized in that** it comprises :
- a direct heating device (12; 42), wherein the viscous fluid is heated up to a temperature range of between 120°C and 155°C by incorporating steam (13) into the viscous fluid;
- a high temperature circuitry (14; 40; 43), fed with heated fluid from the direct heating device (12; 42), wherein the heated fluid is maintained in the temperature range of between 120°C and 155°C for a predetermined residence time, configured to allow sterilization of the viscous fluid; and
- an evaporative cooling device (16) according to any of the claims 1 to 6, fed with the heated fluid from the high temperature circuitry (14; 40; 43), wherein the heated fluid is cooled to a temperature of between 40°C and 90°C by steam evaporation in the vacuum chamber (29) set at a pressure of between 0.05 and 1 bar (absolute pressure).

8. The apparatus according to claim 7, wherein the direct heating device (12) comprises a steam infusion device.

9. The apparatus according to claim 7, wherein the direct heating device (42) comprises a steam injection device.

10. the apparatus according to any of the claims 7 to 9, wherein the high temperature circuitry (14) comprises a centrifugal pump (19) and a relief valve (21), configured to ensure a fluid pressure of between 2 and 10 bars (absolute pressure) upstream of the relief valve (21).

11. the apparatus according to any of the claims 7 to 9, wherein the high temperature circuitry (40) comprises a positive displacement pump (41), configured to ensure a fluid pressure of between 2 and 10 bars (absolute pressure) upstream of the positive displacement pump (41).

12. the apparatus according to any of the claims 7 to 11, wherein the high temperature circuitry (14; 43) comprises a holding tube (20) configured to maintain the heated fluid in a temperature range of between 120°C and 155°C for the predetermined residence time to allow for sterilization of the viscous fluid.

13. the apparatus according to any of the claims 7 to 12, wherein the vacuum level of the vacuum chamber (29) is between 0.05 and 1 bar (absolute pressure).

14. The apparatus according to any of the claims 7 to 13, further comprising an indirect heating device located upstream the direct heating device (12; 42), configured to feed the direct heating device (12; 42) with the viscous fluid at a temperature range of between 40°C and 90°C.

15. An apparatus for the degassing of a viscous fluid comprising an evaporative cooling device (16) according to any of the claims 1 to 6.

16. An apparatus for the concentration of water content of a viscous fluid comprising an evaporative cooling device (16) according to any of the claims 1 to 6.

17. A method for the sterilization of a viscous fluid, wherein the viscous fluid circulates throughout a sterilization circuit comprising an evaporative cooling device (16) according to any of the claims 1 to 6, which method comprises the steps of :
- heating, in less than 1 second, the fluid by incorporation of steam (13) in the fluid, to a temperature range of between 120°C and 155°C;
- maintaining fluid temperature in the range of between 120°C and 155°C for a predetermined residence time, to allow sterilization of the fluid; and
- cooling, in less than 1 second, the fluid by steam evaporation, down to a temperature range of between 40°C and 90°C, in the evaporative cooling device (16).

18. the method according to claim 17 further comprising the step of preheating the fluid, using an indirect heating device, to a temperature of between 40°C and 90°C.

## Patentansprüche

1. Verdampfungs-Kühlvorrichtung für ein viskoses Fluid, Folgendes umfassend:
- eine Vakuumkammer (29), besitzend einen oberen Abschnitt (30), in welchen das viskose Fluid injiziert wird, einen unteren Abschnitt (31), welcher sich abwärts vom oberen Abschnitt (30) erstreckt, worin das viskose Fluid durch Schwerkraft gesammelt wird, und ein Auslassrohr (60), befindlich am Boden des unteren Abschnitts (31), durch welches das viskose Fluid extrahiert wird; und
- eine Extraktionsvorrichtung (17), umfassend eine Primärpumpe (53), gespeist mit viskosem Fluid aus dem unteren Abschnitt (31) durch das Auslassrohr (60), und eine Einspeisevorrichtung (54), befindlich innerhalb des unteren Abschnitts (31) der Vakuumkammer (29), welche das viskose Fluid vom unteren Abschnitt (31) zur Primärpumpe (53) durch das Auslassrohr (60) schiebt,
**dadurch gekennzeichnet, dass**
die Primärpumpe (53) eine Zentrifugalpumpe mit einem Laufrad (61) und einem Antriebsschaft (62) umfasst, wobei die Einspeisevorrichtung (54) einen Propeller (63) umfasst, welcher innerhalb des unteren Abschnitts (31) befindlich ist, und wobei der Antriebsschaft (62) durch das Auslassrohr (60) hindurchführt und mit dem Propeller (63) so verbunden ist, dass er gleichermaßen das Laufrad (61) und den Propeller (63) in Rotation antreibt.

2. Vorrichtung nach Anspruch 1, bei welcher die Einspeisevorrichtung (54) zudem eine Endlosschraube (65) umfasst, verbunden mit dem Antriebsschaft (62), welche sich zwischen dem Laufrad (61) und dem Propeller (63) und mindestens teilweise innerhalb des Auslassrohrs (60) befindet, und wobei der Antriebsschaft (62) in der Lage ist, das Laufrad (61), die Endlosschraube (65) und den Propeller (63) in Rotation anzutreiben.

3. Vorrichtung nach Anspruch 1 oder 2, bei welchem der untere Abschnitt (31) zudem eine konkave Umlauffläche (70) rund um eine im Wesentlichen vertikale Achse umfasst, auf welcher Fläche (70) sich das abgekühlte Fluid ansammelt.

4. Vorrichtung nach einem der vorhergehenden Ansprüche 1 bis 3, bei welcher der Propeller (63) zumindest zwei Blätter (64) umfasst.

5. Vorrichtung nach einem der Ansprüche 1 bis 4, bei welcher die Extraktionsvorrichtung (17) eine Sekundärpumpe (55) umfasst, welche im nachgelagerten Bereich der Primärpumpe (53) befindlich ist, wodurch der Transport des viskosen Fluids von der Primärpumpe (53) zu einem nachgelagerten Kreislauf sichergestellt wird.

6. Vorrichtung nach Anspruch 5, bei welcher die Sekundärpumpe (55) eine Verdrängerpumpe oder eine Zentrifugalpumpe umfasst.

7. Apparat zum Sterilisieren eines viskosen Fluids, **dadurch gekennzeichnet, dass** er Folgendes umfasst:
- eine Direkterhitzungsvorrichtung (12; 42), wobei das viskose Fluid auf einen Temperaturbereich zwischen 120°C und 155°C durch Einarbeiten von Dampf (13) in das viskose Fluid erhitzt wird;
- einen Hochtemperaturkreislauf (14; 40; 43), gespeist mit erhitztem Fluid aus der Direkterhitzungsvorrichtung (12; 42), wobei das erhitzte Fluid in dem Temperaturbereich zwishen 120°C und 155°C für eine vorbestimmte Verweildauer gehalten wird, konfiguriert, um die Sterilisierung des viskosen Fluids zu ermöglichen; und
- eine Verdampfungs-Kühlvorrichtung (16) nach einem der Ansprüche 1 bis 6, gespeist mit dem erhitzten Fluid aus dem Hochtemperaturkreislauf (14; 40; 43), wobei das erhitzte Fluid auf eine Temperatur zwischen 40°C und 90°C durch Dampfverdampfung in der Vakuumkammer (29) abgekühlt wird, welche auf einen Druck zwischen 0,05 und 1 Bar (Absolutdruck) eingestellt ist.

8. Apparat nach Anspruch 7, bei welchem die Direkterhitzungsvorrichtung (12) eine Dampfinfusionsvorrichtung umfasst.

9. Apparat nach Anspruch 7, bei welchem die Direkterhitzungsvorrichtung (42) eine Dampfinjektionsvorrichtung umfasst.

10. Apparat nach einem der Ansprüche 7 bis 9, bei welchem der Hochtemperaturkreislauf (14) eine Zentrifugalpumpe (19) und ein Entlastungsventil (21) umfasst, konfiguriert zur Sicherstellung eines Fluiddrucks zwischen 2 und 10 Bar (Absolutdruck) im vorgelagerten Bereich des Entlastungsventils (21).

11. Apparat nach einem der Ansprüche 7 bis 9, bei welchem der Hochtemperaturkreislauf (40) eine Verdrängerpumpe (41) umfasst, konfiguriert zur Sicherstellung eines Fluiddrucks zwischen 2 und 10 Bar (Absolutdruck) im vorgelagerten Bereich der Verdrängerpumpe (41).

12. Apparat nach einem der Ansprüche 7 bis 11, bei welchem der Hochtemperaturkreislauf (14; 43) ein Halterohr (20) umfasst, konfiguriert zur Haltung des erhitzten Fluids in einem Temperaturbereich zwischen 120°C und 155°C für die vorbestimmte Verweildauer, um die Sterilisierung des viskosen Fluids zu ermöglichen.

13. Apparat nach einem der Ansprüche 7 bis 12, bei welchem das Vakuumniveau der Vakuumkammer (29) zwischen 0,05 und 1 Bar (Absolutdruck) beträgt.

14. Apparat nach einem der Ansprüche 7 bis 13, zudem umfassend eine indirekte Erhitzungsvorrichtung, befindlich im vorgelagerten Bereich der Direkterhitzungsvorrichtung (12; 42), konfiguriert zum Speisen der Direkterhitzungsvorrichtung (12; 42) mit dem viskosen Fluid in einem Temperaturbereich zwischen 40°C und 90°C.

15. Apparat zum Entgasen eines viskosen Fluids, umfassend eine Verdampfungs-Kühlvorrichtung (16) nach einem der Ansprüche 1 bis 6.

16. Apparat zum Aufkonzentrieren des Wassergehaltes eines viskosen Fluids, umfassend eine Verdampfungs-Kühlvorrichtung (16) nach einem der Ansprüche 1 bis 6.

17. Verfahren zum Sterilisieren eines viskosen Fluids, bei welchem das viskose Fluid einen Sterilisationskreislauf durchläuft, umfassend eine Verdampfungs-Kühlvorrichtung (16) nach einem der Ansprüche 1 bis 6, wobei das Verfahren die folgenden Schritte umfasst:
- Erhitzen, in weniger als 1 Sekunde, des Fluids durch Einarbeiten von Dampf (13) in das Fluid, auf einen Temperaturbereich zwischen 120°C und 155°C;
- Halten der Fluidtemperatur in dem Bereich zwischen 120°C und 155°C über eine vorbestimmte Verweildauer, um die Sterilisierung des Fluids zu ermöglichen; und
- Abkühlen, in weniger als 1 Sekunde, des Fluids durch Verdampfen von Dampf, auf einen Temperaturbereich zwischen 40°C und 90°C in der Verdampfungs-Kühlvorrichtung (16).

18. Verfahren nach Anspruch 17, zudem umfassend den Schritt des Vorwärmens des Fluids unter Verwendung einer indirekten Erhitzungsvorrichtung auf eine Temperatur zwischen 40°C und 90°C.

## Revendications

1. Dispositif de refroidissement par évaporation destiné à un fluide visqueux, comprenant :
- une chambre à vide (29), présentant une partie supérieure (30) au sein de laquelle est injecté le fluide visqueux, une partie inférieure (31) s'étendant vers le bas à partir de la partie supérieure (30) et au sein de laquelle le fluide visqueux est collecté grâce à la force de gravitation, et une conduite de refoulement (60) située au bas de la partie inférieure (31) et par l'intermédiaire de laquelle est extrait le fluide visqueux ; et
- un dispositif d'extraction (17), comprenant une pompe primaire (53), alimentée avec du fluide visqueux en provenance de la partie inférieure (31) par l'intermédiaire de la conduite de refoulement (60), et un dispositif d'alimentation (54), situé à l'intérieur de la partie inférieure (31) de la chambre à vide (29), qui pousse le fluide visqueux en provenance de la partie inférieure (31) en direction de la pompe primaire (53), en passant par la conduite de refoulement (60),
**caractérisé en ce que**
la pompe primaire (53) comprend une pompe centrifuge présentant un rotor (61) et un arbre de transmission (62), dans lequel le dispositif d'alimentation (54) comprend une hélice (63) située à l'intérieur de la partie inférieure (31), et dans lequel l'arbre de transmission (62) passe à travers la conduite de refoulement (60) et est lié à l'hélice (63) de manière à entraîner en rotation à la fois le rotor (61) et l'hélice (63).

2. Dispositif selon la revendication 1, dans lequel le dispositif d'alimentation (54) comprend en outre une vis sans fin (65) liée à l'arbre de transmission (62), située entre le rotor (61) et l'hélice (63), et se trouvant au moins partiellement à l'intérieur de la conduite de refoulement (60), et dans lequel l'arbre de transmission (62) est en mesure d'entraîner en rotation le rotor (61), la vis sans fin (65) et l'hélice (63).

3. Dispositif selon la revendication 1 ou 2, dans lequel la partie inférieure (31) comprend en outre une surface de révolution concave (70) autour d'un axe essentiellement vertical, le fluide refroidi s'accumulant sur ladite surface (70).

4. Dispositif selon l'une quelconque des revendications 1 à 3, dans lequel l'hélice (63) comprend au moins deux pales (64).

5. Dispositif selon l'une quelconque des revendications 1 à 4, dans lequel le dispositif d'extraction (17) comprend une pompe secondaire (55) située en aval de la pompe primaire (53) et qui assure le transport du fluide visqueux de la pompe primaire (53) vers un circuit aval.

6. Dispositif selon la revendication 5, dans lequel la pompe secondaire (55) comprend une pompe volumétrique ou une pompe centrifuge.

7. Appareil destiné à la stérilisation d'un fluide visqueux, **caractérisé en ce qu'**il comprend :
- un dispositif de chauffage direct (12 ; 42), dans lequel le fluide visqueux est chauffé jusqu'à une plage de température comprise entre 120°C et 155°C grâce à une étape consistant à incorporer de la vapeur (13) dans le fluide visqueux ;
- un circuit haute température (14 ; 40 ; 43), alimenté avec du fluide chauffé en provenance du dispositif de chauffage direct (12 ; 42), dans lequel le fluide chauffé est maintenu à l'intérieur de la plage de température comprise entre 120°C et 155°C pendant un temps de séjour prédéterminé, configuré pour permettre la stérilisation du fluide visqueux ; et
- un dispositif de refroidissement par évaporation (16) selon l'une quelconque des revendications 1 à 6, alimenté avec le fluide chauffé en provenance du circuit haute température (14 ; 40 ; 43), dans lequel le fluide chauffé est refroidi à une température comprise entre 40°C et 90°C grâce à une évaporation de vapeur dans la chambre à vide (29) ajustée à une pression comprise entre 0,05 et 1 bar (pression absolue).

8. Appareil selon la revendication 7, dans lequel le dispositif de chauffage direct (12) comprend un dispositif à infusion de vapeur.

9. Appareil selon la revendication 7, dans lequel le dispositif de chauffage direct (42) comprend un dispositif à injection de vapeur.

10. Appareil selon l'une quelconque des revendications 7 à 9, dans lequel le circuit haute température (14) comprend une pompe centrifuge (19) et une soupape de surpression (21), configurée pour assurer une pression de fluide comprise entre 2 et 10 bars (pression absolue) en amont de la soupape de surpression (21).

11. Appareil selon l'une quelconque des revendications 7 à 9, dans lequel le circuit haute température (40) comprend une pompe volumétrique (41), configurée pour assurer une pression de fluide comprise entre 2 et 10 bars (pression absolue) en amont de la pompe volumétrique (41).

12. Appareil selon l'une quelconque des revendications 7 à 11, dans lequel le circuit haute température (14 ; 43) comprend un tube chambreur (20) configuré pour maintenir le fluide chauffé à l'intérieur d'une plage de température comprise entre 120°C et 155°C pendant le temps de séjour prédéterminé afin de permettre la stérilisation du fluide visqueux.

13. Appareil selon l'une quelconque des revendications 7 à 12, dans lequel le niveau de vide de la chambre à vide (29) est compris entre 0,05 et 1 bar (pression absolue).

14. Appareil selon l'une quelconque des revendications 7 à 13, comprenant en outre un dispositif de chauffage indirect situé en amont du dispositif de chauffage direct (12 ; 42), configuré pour alimenter le dispositif de chauffage direct (12 ; 42) avec le fluide visqueux à l'intérieur d'une plage de température comprise entre 40°C et 90°C.

15. Appareil destiné au dégazage d'un fluide visqueux, comprenant un dispositif de refroidissement par évaporation (16) selon l'une quelconque des revendications 1 à 6.

16. Appareil destiné à la concentration de la teneur en eau d'un fluide visqueux, comprenant un dispositif de refroidissement par évaporation (16) selon l'une quelconque des revendications 1 à 6.

17. Procédé de stérilisation d'un fluide visqueux, dans lequel le fluide visqueux circule à travers un circuit de stérilisation comprenant un dispositif de refroidissement par évaporation (16) selon l'une quelconque des revendications 1 à 6, ledit procédé comprenant les étapes consistant à :
- chauffer, en moins d'une seconde, le fluide par incorporation de vapeur (13) dans ledit fluide, jusqu'à une plage de température comprise entre 120°C et 155°C ;
- maintenir la température du fluide à l'intérieur de la plage comprise entre 120°C et 155°C pendant un temps de séjour prédéterminé, afin de permettre la stérilisation du fluide ; et
- refroidir, en moins d'une seconde, le fluide par évaporation de la vapeur, jusqu'à une plage de température comprise entre 40°C et 90°C, au sein du dispositif de refroidissement par évaporation (16).

18. Procédé selon la revendication 17, comprenant en outre l'étape consistant à préchauffer le fluide, en utilisant un dispositif de chauffage indirect, jusqu'à une température comprise entre 40°C et 90°C.
